# EUROPEAN PATENT APPLICATION

(11) **EP 2 518 507 A1**
(43) Date of publication of application: **31.10.2012**
(21) Application number: 12166346.2
(22) Date of filing: 19.05.2008
(51) Int. Cl.: G01N 33/574

(54) **Serum biomarkers for the early detection of lung cancer**

(30) Priority: 18.05.2007 US 930886 P; 16.08.2007 US 965053 P
(62) Divisional of application: 08767809.0
(71) Applicant: Duke University, Durham, North Carolina 27708-0083 (US)
(72) Inventor: Patz, Edward, F., Jr., Chapel Hill, NC 27517 (US); Campa, Michael J., Durham, NC 27701 (US)
(74) Representative: Isarpatent

(57) **Abstract**

Methods and kits are provided for determining the probability of lung cancer, for managing treatment of subjects with potential lung cancer and for molecular staging of lung tumors. The methods include determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; and assigning the subject to a group having a higher or lower probability of lung cancer based on the determined amount of each biomarker the panel.

## Description

### CROSS-REFERENCE TO RELATED APPLICATIONS

This application embodiments benefit of U.S. Provisional Application, Serial No. 60/965,053, titled "Serum Biomarkers for the Early Detection of Lung Cancer," filed August 16, 2007, and U.S. Provisional Application, Serial No. 60/930,886, titled "Novel Serum Biomarkers for the Detection of Lung Cancer," filed May 18, 2007, both of which are herein incorporated by reference in their entireties.

### TECHNICAL FIELD

The presently disclosed subject matter pertains to the use of biomarkers in the detection of lung cancer.

### BACKGROUND

Lung cancer continues to be a significant worldwide public health issue. Although advances in noninvasive imaging have improved the ability to detect lung cancer, >75% of lung cancer patients present with advanced stage disease when therapeutic options are limited (1). Even those patients who present with clinical stage I lung cancer have at best a 60% 5-year survival rate, signifying that a large percentage of all stage I patients have undetectable metastatic disease at the time of presentation (1). These statistics underscore the need for improvements in early detection strategies and more accurate molecular staging of tumors.

Lung cancer accounts for more cancer deaths than any other malignancy. Despite advances in diagnostic capabilities and treatment, lung cancer mortality has not significantly changed over the past several decades. Most patients present with inoperable disease when therapeutic options including chemotherapy and radiotherapy are rarely curative. Screening studies for lung cancer with chest radiographs and sputum cytology have failed to show that this approach will decrease the number of patients that die from the disease (16).

More recent trials with low-dose spiral computed tomography (CT) have found it can detect small tumors, but no study has shown that this will reduce lung cancer mortality, the ultimate goal of a screening program (17). These trials also illustrate a number of challenging issues with regard to imaging as a solitary early detection strategy for lung cancer.

First, it has been recommended that all high-risk individuals (i.e., smokers and former smokers) be screened by CT (2, 18). However, only a small percentage of individuals currently classified as high-risk develop lung cancer, and even some of these cancers can have an indolent phenotype; i.e., there is an overdiagnosis bias (3, 17, 19, 20). Thus, defining the optimal population to be screened remains unresolved. Second, the high false positive rate in CT screening trials dictates that a large number of individuals undergo follow-up studies. More troublesome is that in some CT screening trials, up to 30% of resected nodules were benign. Thus, a number of individuals underwent unnecessary thoracotomy, with all of the morbidity and mortality associated with this procedure (3). While several strategies to evaluate indeterminate lesions have been suggested, there is no evidence-based consensus as to the appropriate manner for management of these patients.

Accordingly, there remains an unmet need for approaches that provide for the early detection and molecular staging of tumors.

### SUMMARY

Methods and kits are provided for determining the probability of lung cancer, for managing treatment of subjects with potential lung cancer and for molecular staging of lung tumors. In some embodiments, a method is provided for assigning a subject to a group having a higher or lower probability of lung cancer, comprising, determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; and assigning the subject to a group having a higher or lower probability of lung cancer based on the determined amount of each biomarker the panel. In some embodiments, the sample is a serum sample. In some embodiments, the subject is a human subject.

In some embodiments, the panel of biomarkers comprises at least three of the proteins alpha-1-antitrypsin, carcinoembryonic antigen, squamous cell carcinoma antigen, retinol binding protein, transferrin and haptoglobin. In some embodiments, the panel of biomarkers comprises at least four of the proteins alpha-1-antitrypsin, carcinoembryonic antigen, squamous cell carcinoma antigen, retinol binding protein, transferrin and haptoglobin. In some embodiments, the panel of biomarkers comprises at least the proteins alpha-1-antitrypsin, carcinoembryonic antigen, squamous cell carcinoma antigen and retinol binding protein. In some embodiments, the panel of biomarkers comprises at least the proteins alpha-1-antitrypsin, carcinoembryonic antigen, squamous cell carcinoma antigen, retinol binding protein, transferrin, and haptoglobin.

In some embodiments, the method comprises applying a predetermined algorithm to the determined amount of each biomarker in the panel; and employing the results of the algorithm to assign the subject to the group having a higher or lower probability of lung cancer. In some embodiments, the algorithm is a decision tree analysis. In some embodiments, the algorithm is a Classification and Regression Tree analysis.

In some embodiments, a method is provided for managing treatment of a subject with potential lung cancer, comprising, determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; assigning the subject to a group having a higher or lower probability of lung cancer based on the determined amount of each biomarker the panel; and managing the treatment of the subject with potential lung cancer based on the group to which the subject is assigned.

In some embodiments, a method is provided for molecular staging of a lung tumor or suspected lung tumor, comprising, determining an amount of each member of a panel of biomarkers in a sample from a subject having a tumor or a suspected tumor, wherein the panel of biomarkers comprises at least two of the proteins alpha-1-antitrypsin, carcinoembryonic antigen, squamous cell carcinoma antigen, retinol binding protein, transferrin, or haptoglobin; assigning the subject to a group having a higher or lower probability of a stage of lung tumor or suspected lung tumor based on the determined amount of each biomarker in each panel; and determining the molecular stage of the tumor or suspected tumor based on the group to which the subject is assigned.

In some embodiments, a method is provided for assigning a subject to a high-risk group for lung cancer comprising, determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; and assigning the subject to a group having a high-risk of lung cancer based on the determined amount of each biomarker in the panel.

In some embodiments, a kit is provided for determining the probability of the presence of lung cancer in a subject based on measuring an amount of each member of a panel of biomarkers in a sample of the subject, the kit comprising: detection molecules specific for each of the biomarkers in the panel, wherein the biomarkers comprise at least two of the proteins selected from the group consisting of alpha-1-antitrypsin, carcinoembryonic antigen, squamous cell carcinoma antigen, retinol binding protein, transferrin, and haptoglobin; and directions for measuring the amount of each member of the panel of biomarkers and determining the probability of lung cancer. In some embodiments, the sample is a serum sample from a human subject.

In some embodiments of the kit, the detection molecules comprise a conjugated detectable group. In some embodiments, the detection molecules are immobilized on a solid support. In some embodiments, the detection molecules comprise antibodies specific for each of the protein biomarkers in the panel. In some embodiments, the kit comprises a second specific antibody for each of the antibodies specific for the protein biomarkers, wherein the second specific antibody is conjugated to a detectable group. In some embodiments, the kit comprises a specific binding partner for each of the detection molecules specific for the biomarkers in the panel, wherein each specific binding partner is conjugated to a detectable group. In some embodiments, the detectable group is selected from the group consisting of radioactive labels, fluorescent labels, enzyme labels and fluorescent labels. In some embodiments, the kit comprises one or more of buffering agents, protein stabilizing agents, enzyme substrates, background reducing agents, control reagents, an apparatus for conducting the detection, and any necessary software for analysis and presentation of results.

In some embodiments of the kit, the biomarkers comprise at least three of the proteins selected from the group consisting of alpha-1-antitrypsin, carcinoembryonic antigen, squamous cell carcinoma antigen, retinol binding protein, transferrin, and haptoglobin. In some embodiments, the biomarkers comprise at least four of the proteins selected from the group consisting of alpha-1-antitrypsin, carcinoembryonic antigen, squamous cell carcinoma antigen, retinol binding protein, transferrin, and haptoglobin. In some embodiments, the biomarkers comprise at least the proteins alpha-1-antitrypsin, carcinoembryonic antigen, squamous cell carcinoma antigen and retinol binding protein. In some embodiments, the biomarkers comprise at least the proteins alpha-1-antitrypsin, carcinoembryonic antigen, squamous cell carcinoma antigen, retinol binding protein, transferrin, and haptoglobin.

Accordingly, it is an object of the presently disclosed subject matter to provide a novel method for detecting lung cancer. This and other objects are achieved in whole or in part by the presently disclosed subject matter.

An object of the presently disclosed subject matter having been stated above, other objects and advantages will become apparent upon a review of the following descriptions, figures and examples.

### BRIEF DESCRIPTION OF THE DRAWINGS

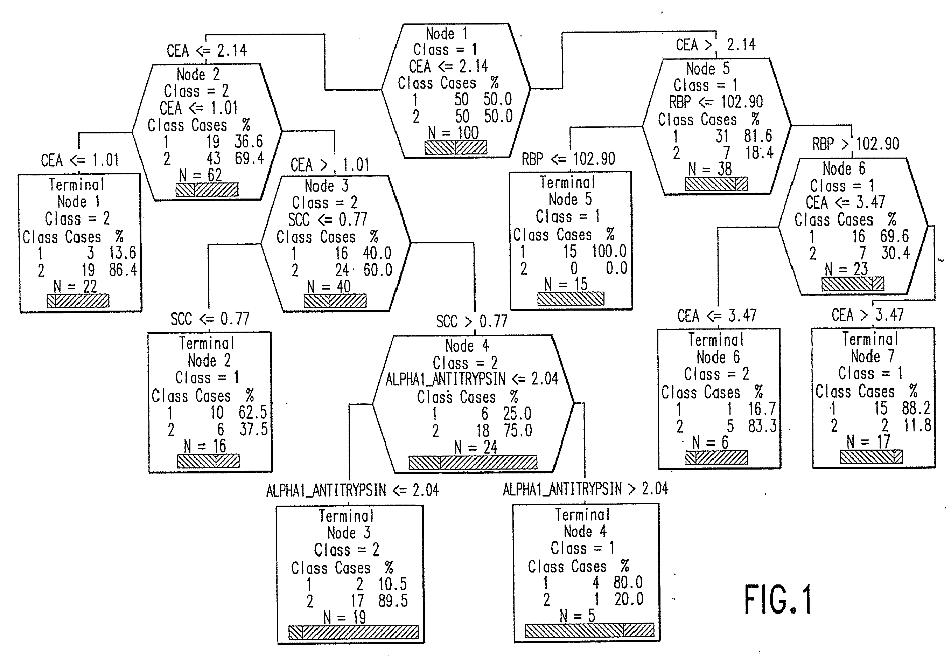

Figure 1 is a graphical depiction of the results of Classification and Regression Tree analysis (CART) analysis of a Training Set of 100 serum specimens as being from individuals with (Class 1) or without (Class 2) lung cancer. The analysis resulted in selection of 4 protein biomarkers, carcinoembryonic antigen (CEA), retinol binding protein (RBP), squamous cell carcinoma antigen (SCC), and alpha-1 antitrypsin (AAT), with 7 terminal nodes. In Figure 1, the Class 1 samples are represented as forward slash hatching and the Class 2 samples are represented as backward slash hatching. Overall, the tree correctly classified 44/50 (88%) of lung cancer sera and 41/50 (82%) of non-cancer sera (sensitivity 89.3%, specificity 84.7%). Sixty-eight percent (34/50) of all lung cancer cases were binned in terminal nodes 4, 5 and 7, while only 6% (3/50) of control cases were in these nodes. Therefore if a patient was assigned to one of these terminal nodes, there was a 92% (34/37) probability the patient had lung cancer.

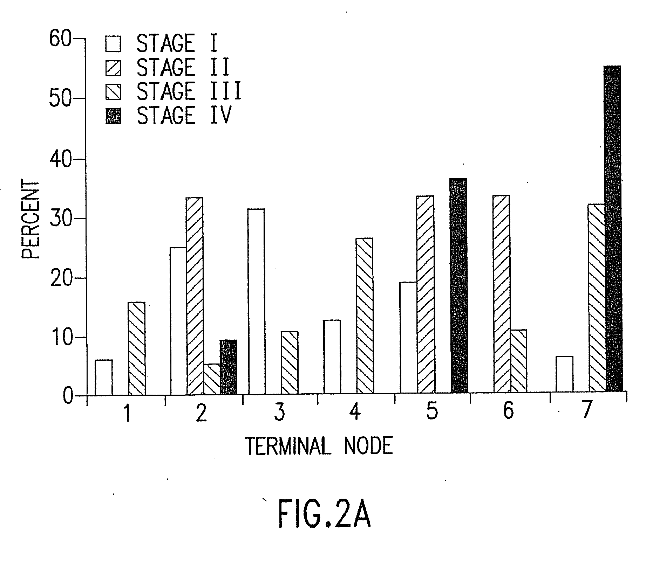

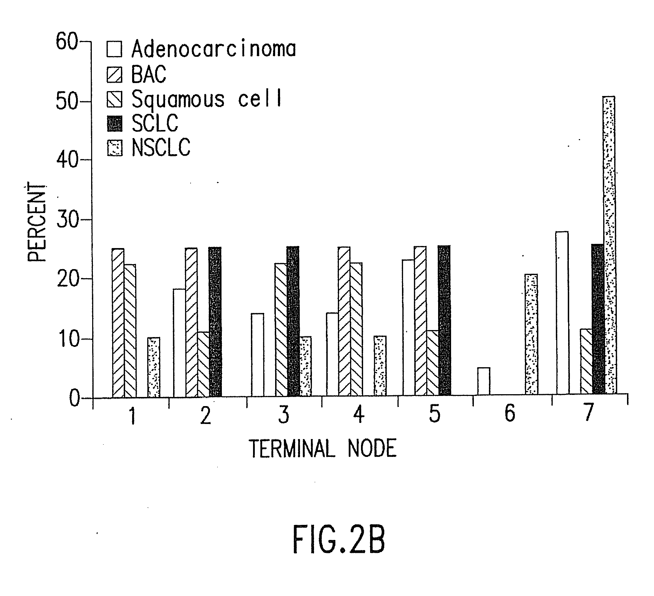

Figures 2A and 2B are bar graphs showing the percent cancer by stage (Panel A) and type (Panel B) in each of the 7 terminal nodes resulting from the CART analysis of a test set of sera. The classification tree derived in Figure 1 was tested on a blinded, independent set of biomarker data from serum samples of 49 patients with a new diagnosis of lung cancer and 48 age and gender matched controls. In both Panel A and B, the percent represented on the y-axis refers to the percent of all cancers of a specific stage or histology, respectively, that appear in each of the 7 terminal nodes. Sixty-two percent (10/16) of stage I patients, 66% (2/3) of stage II patients, 63% (12/19) of stage III patients, and 100% of stage IV patients (11/11) were accurately assigned to a lung cancer node, as shown in **Figure 2A**. In Figure 2A, Stage I is represented as an open box; Stage II is represented as forward slash hatching; Stage III is represented as backward slash hatching; and Stage IV is represented as a filled box. The distribution of histology according to the terminal nodes is shown in **Figure 2B**. In Figure 2B, Adenocarcinoma is represented as an open box; BAC is represented as forward slash hatching; Squamous cell is represented as backward slash hatching; SCLC is represented as a filled box; NSCLC is represented as stippling.

### DETAILED DESCRIPTION

In accordance with the presently disclosed subject matter, methods and compositions are provided for the detection, desirably early detection, and molecular staging of tumors. A panel of biomarkers is provided for the detection of lung cancer. The presently provided biomarkers can define high-risk patients and can suggest which patients with indeterminate pulmonary nodules have lung cancer. The biomarkers enhance diagnostic capabilities, complement imaging studies, and have clinical benefit for detection of cancer.

Unless otherwise defined, all technical and scientific terms used herein have the same meaning as commonly understood by one of ordinary skill in the art to which this presently described subject matter belongs. All publications, patent applications, patents, and other references mentioned herein are incorporated by reference in their entirety.

### I. DEFINITIONS

While the following terms are believed to be well understood by one of ordinary skill in the art, the following definitions are set forth to facilitate explanation of the presently disclosed subject matter.

Following long-standing patent law convention, the terms "a" and "an" mean "one or more" when used in this application, including the embodiments.

Unless otherwise indicated, all numbers expressing quantities of ingredients, reaction conditions, and so forth used in the specification and embodiments are to be understood as being modified in all instances by the term "about". Accordingly, unless indicated to the contrary, the numerical region of parameters set forth in this specification and attached embodiments are approximations that can vary depending upon the desired properties sought to be obtained by the presently disclosed subject matter.

"Amino acid sequence" and terms such as "peptide", "polypeptide" and "protein" are used interchangeably herein, and are not meant to limit the amino acid sequence to the complete, native amino acid sequence (i.e. a sequence containing only those amino acids found in the protein as it occurs in nature) associated with the recited protein molecule. The proteins and protein fragments of the presently disclosed subject matter can be produced by recombinant approaches or can be isolated from a naturally occurring source. The protein fragments can be any size, and for example can range in size from four amino acid residues to the entire amino acid sequence minus one amino acid.

The term "antibody" includes any antibody fragments that bind with sufficient specificity to a protein of interest.

The phrase "detection molecule" is used herein in its broadest sense to include any molecule that can bind with sufficient specificity to one of the members of the biomarker panel to allow for detection of the particular biomarker member in the presence or absence of the other members of the panel. To allow for detection can mean to determine the presence or absence of the particular biomarker member and, in some embodiments, can mean to determine the amount of the particular biomarker. Detection molecules can include antibodies and antibody fragments.

As used herein, the term "sample" is used in its broadest sense. In one sense, it is meant to include a specimen from a biological source. Biological samples can be obtained from animals (including humans) and encompass fluids, solids, tissues, and gases. Biological samples include blood products, such as plasma, serum and the like.

The phrase "a specific binding partner for each of the detection molecules" is used herein to include any molecule that binds with sufficient specificity to one of the detection molecules to allow for detection of the particular detection molecule in the presence or absence of the detection molecules for the other members of the biomarker panel. For example, in some embodiments the specific binding partner can be a secondary antibody that recognizes the detection molecule that is a primary antibody. In some embodiments the specific binding partner can be a molecule that specifically binds to a group on the detection molecule such as, for example, a biotin group on the detection molecule.

As used herein, the term "subject" refers to any animal (e.g., a mammal), including, but not limited to, humans, non-human primates, rodents, and the like, which is to be the recipient of a particular treatment. The terms "subject" and "patient" are used interchangeably herein, such as but not limited to in reference to a human subject.

As used herein, the term "subject suspected of having lung cancer" refers to a subject that presents one or more symptoms indicative of lung cancer or is being screened for lung cancer (e.g., during a routine physical). A subject suspected of having lung cancer can also have one or more risk factors. A subject suspected of having cancer has generally not been tested for cancer. However, a "subject suspected of having cancer" can also encompass an individual who has received an initial diagnosis (e.g., a CT scan showing an indeterminate pulmonary nodule) but for whom the stage of cancer is not known. The term further includes people who once had lung cancer (e.g., an individual in remission).

As used herein, the term "subject at risk for cancer" refers to a subject with one or more risk factors for developing lung cancer.

### II. REPRESENTATIVE EMBODIMENTS

The presently disclosed subject matter provides a panel of serum biomarkers useful for the detection, desirably early detection, of lung cancer and the molecular staging of tumors. In some aspects, the panel of serum biomarkers provided herein addresses certain limitations of early detection of tumors by CT screening alone.

The concept of biomarkers is founded on the biological properties of cancer as a systemic disease. As a malignancy develops, it secretes proteins required for growth and metastasis and sheds cells into the circulation. The host responds by inducing changes in tissue architecture and vasculature in the microenvironment of the incipient tumor, as well as systemically mounting an immunological defense. This can include innate and adaptive responses with migration of inflammatory cells including macrophages, histiocytes and lymphocytes into the tumor, and the production of autoantibodies (21-24). Thus, a combination of tumor-expressed and host response proteins, if identified, can be useful to develop a profile of cancer for clinical screening.

Since lung cancer is a heterogeneous disease, a panel of markers that covers the broad clinical phenotype is needed. Transferrin, retinol binding protein (RBP), and haptoglobin were identified from a 2-dimentional difference gel electrophoresis (2D-DIGE) experiment and alpha-1 antitrypsin (AAT) from a Matrix-assisted laser desorption/ionization-time of flight mass spectrometry (MALDI-TOF MS) experiment (see Example 1). This group of biomarkers was supplemented with two proteins, carcinoembryonic antigen (CEA) and squamous cell carcinoma antigen (SCC), whose serum levels are known to vary depending on lung cancer status, but have insufficient sensitivity and specificity as stand-alone diagnostic assays or in combination with a variety of other proteins (11, 14, 15) (see Example 1).

Seven proteins were initially assayed. Classification and Regression Tree analysis (CART) analysis of a training set indicated that four markers in particular - CEA, RBP, SCC, and AAT - are sufficient to classify 88% of patients with cancer and 82% of patients without cancer correctly (see Examples 4-5). The classification scheme produced by CART analysis assigns each patient a probability of malignancy based on the terminal node into which he or she falls. See Figure 1. In the training set, if a patient fell into one of three terminal nodes - nodes 4, 5 or 7 of Figure 1 - there was a 92% chance that the patient had lung cancer. In the validation phase of the study, the classification tree correctly classified 71.4% of the lung cancer patients and 66.6% of the non-cancer control patients (see Example 6). Again, nodes 4, 5 and 7 of Figure 1 provide a representative lung cancer prediction scheme: 57% of all lung cancer cases were binned in these nodes, while only 6% of control cases were in these nodes. If a patient was assigned to one of these terminal nodes, the patient had a 90% chance of having lung cancer.

In combination, the presently disclosed protein markers provide significant clinical utility for the early detection of lung cancer and the molecular staging of tumors. Accordingly, in some embodiments methods are provided for assigning a subject to a group having a higher or lower probability of lung cancer. In some embodiments, the methods comprise: determining the level of each of a panel of biomarkers in a serum sample from the patient, wherein the panel of biomarkers comprises at least two of the proteins alpha-1-antitrypsin, carcinoembryonic antigen, squamous cell carcinoma antigen, retinol binding protein, transferrin, or haptoglobin; and assigning the patient to the group having a higher or lower probability of lung cancer based on the determined amount of each biomarker in the panel.

In some embodiments, a method is provided for assigning a subject to a high-risk group for lung cancer. The method can comprise: determining the level of each of a panel of biomarkers in a serum sample from the subject, wherein the panel of biomarkers comprises at least two of the proteins alpha-1-antitrypsin, carcinoembryonic antigen, squamous cell carcinoma antigen, retinol binding protein, transferrin or haptoglobin; and determining whether the subject should be assigned to the group having a high-risk of lung cancer based on the determined amount of each biomarker in the panel.

In some embodiments, a method is provided for managing treatment of a subject with potential lung cancer. The method can comprise: determining the level of each of a panel of biomarkers in a serum sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; assigning the subject to a group having a higher or lower probability of lung cancer based on the determined amount of each biomarker in the panel; and managing the treatment of the subject with potential lung cancer based on the group to which the subject is assigned.

In some embodiments, a method is provided for molecular staging of a tumor or suspected tumor. The method can comprise: determining the level of each of a panel of biomarkers in a serum sample from a subject having a tumor or suspected tumor, wherein the panel of biomarkers comprises at least two of the proteins alpha-1-antitrypsin, carcinoembryonic antigen, squamous cell carcinoma antigen, retinol binding protein, transferrin, or haptoglobin; assigning the subject to a group having a higher or lower probability of a stage of lung tumor or suspected lung tumor based on the determined amount of each biomarker in the panel; and determining the stage of the tumor or suspected tumor based on the group to which the subject is assigned.

The level of each of the presently disclosed panel of biomarkers can be determined in a variety of animal tissues. In some embodiments, the biomarkers can be detected in animal tissue or bodily fluids. In some embodiments, the biomarkers can be detected in bodily fluids including plasma, serum, whole blood, mucus, and/or urine. In a particular embodiment, the biomarkers can be detected in serum.

In some embodiments, the presently disclosed methods can comprise statistically analyzing the amounts of each biomarker. The statistical analysis can comprise applying a predetermined algorithm to the amounts of the biomarkers. The results of the algorithm can be employed to assign a subject to a group having a higher or lower probability of lung cancer.

A variety of algorithms can be employed in the presently disclosed methods. The algorithms employed are not limited to those described in the Examples herein, but rather include algorithms as would be apparent to those of ordinary skill in the art upon a review of the instant disclosure. In some embodiments, the algorithm employed can be a decision tree analysis. In some embodiments, the algorithm employed can be a Classification and Regression Tree analysis (CART).

The level of each of a panel of biomarkers can be determined in the presently disclosed methods. In some embodiments, the panel of biomarkers can comprise at least three of the proteins alpha-1-antitrypsin, carcinoembryonic antigen, squamous cell carcinoma antigen, retinol binding protein, transferrin and haptoglobin. In some embodiments, the panel of biomarkers can comprise at least four of the proteins alpha-1-antitrypsin, carcinoembryonic antigen, squamous cell carcinoma antigen, retinol binding protein, transferrin and haptoglobin. In some embodiments, the panel of biomarkers can comprise at least the proteins alpha-1-antitrypsin, carcinoembryonic antigen, squamous cell carcinoma antigen and retinol binding protein. In some embodiments, the panel of biomarkers can comprise at least the proteins alpha-1-antitrypsin, carcinoembryonic antigen, squamous cell carcinoma antigen, retinol binding protein, transferrin, and haptoglobin.

The presently disclosed subject matter is not limited to the panel of biomarkers described above. Any marker that correlates with lung cancer or the progression of lung cancer can be included in the biomarker panel provided herein, and is within the scope of the presently disclosed subject matter. Any suitable method can be utilized to identify additional lung cancer biomarkers suitable for use in the presently disclosed methods, including but not limited to, the methods described in the illustrative Examples below. For example, biomarkers that are known or identified as being up or downregulated in lung cancer using methods known to those of ordinary skill in the art can be employed. Additional biomarkers can include one or more of polypeptides, small molecule metabolites, lipids and nucleotide sequences. Markers for inclusion on a panel can be selected by screening for their predictive value using any suitable method, including but not limited to, those described in the illustrative Examples below.

As is apparent from the foregoing embodiments, the presently disclosed methods and compositions are useful for screening patients for lung cancer, for the early detection of lung cancer, and for managing the treatment of patients with potential lung cancer or with known lung cancer. For example, in some embodiments, the panel of biomarkers can be useful for screening patients prior to imaging or other known methods for detecting lung tumors, to define patients at high risk or higher risk for lung cancer. In this manner, only those patients with both a high risk clinical profile and a test result from the biomarker panel indicating a high or higher probability of lung cancer can be sent on to have a CT scan performed. Patients whose test results suggest a low probability of cancer can be reevaluated using the panel of serum biomarkers during their routine follow-up.

In some embodiments, the panel of serum biomarkers provided herein can be employed where an indeterminate pulmonary nodule is detected on imaging studies, whether detected in a screening trial or performed for other indications. Those patients with a test result from the biomarker panel indicating a low risk of lung cancer can be followed with imaging studies at intervals dictated by the risk probability of their terminal node test result. Patients having both a high-risk clinical profile and a terminal node associated with a high risk of malignancy can be determined to require immediate intervention. As an illustrative example, in the study described in Examples 5-6 herein below, over 60% of lung cancer patients fell into one of three malignant terminal nodes with a >90% probability of cancer (see Figure 1 and Table 2). In these patients a positron emission tomography (PET) scan can then be performed, followed by surgery or a biopsy, depending on other clinical factors.

Any suitable method can be employed for determining the level of each of the panel of biomarkers, as would be apparent to one skilled in the art upon a review of the present disclosure. For example, methods for detecting biomarkers can include gas chromatography (GC), liquid chromatography/mass spectroscopy (LC-MS), gas chromatography/mass spectroscopy (GC-MS), nuclear magnetic resonance (NMR), magnetic resonance imaging (MRI), Fourier Transform InfraRed (FT-IR), and inductively coupled plasma mass spectrometry (ICP-MS). It is further understood that mass spectrometry techniques include, but are not limited to, the use of magnetic-sector and double focusing instruments, transmission quadrapole instruments, quadrupole ion-trap instruments, time-of-flight instruments (TOF), Fourier transform ion cyclotron resonance instruments (FT-MS), and matrix-assisted laser desorption/ionization time-of-flight mass spectrometry (MALDI-TOF MS).

In some embodiments, protein biomarkers can be detected using technologies well known to those of skill in the art such as gel electrophoresis, immunohistochemistry, and antibody binding. Methods for generating antibodies to a polypeptide of interest are well known to those of ordinary skill in the art. An antibody against a protein biomarker of the presently disclosed subject matter can be any monoclonal or polyclonal antibody, so long as it suitably recognizes the protein biomarker. In some embodiments, antibodies are produced using the protein biomarker as the immunogen according to any conventional antibody or antiserum preparation process. The presently disclosed subject matter provides for the use of both monoclonal and polyclonal antibodies. In addition, a protein used herein as the immunogen is not limited to any particular type of immunogen. For example, fragments of the protein biomarkers of the presently disclosed subject matter can be used as immunogens. The fragments can be obtained by any method including, but not limited to, expressing a fragment of the gene encoding the protein, enzymatic processing of the protein, chemical synthesis, and the like.

The antibodies of the presently disclosed subject matter can be useful for detecting the protein biomarkers. For example, antibody binding is detected by techniques known in the art (e.g., radioimmunoassay, ELISA (enzyme-linked immunosorbant assay), "sandwich" immunoassays, immunoradiometric assays, gel diffusion precipitation reactions, immunodiffusion assays, *in situ* immunoassays (e.g., using colloidal gold, enzyme or radioisotope labels, for example), Western blots, precipitation reactions, agglutination assays (e.g., gel agglutination assays, hemagglutination assays, etc.), complement fixation assays, immunofluorescence assays, protein A assays, and immunoelectrophoresis assays, etc. One example of an immunoassay is described in U.S. Pat. Nos. 5,599,677 and 5,672,480, each of which is herein incorporated by reference. Upon review of the present disclosure, those skilled in the art will be familiar with numerous specific immunoassay formats and variations thereof that can be useful for carrying out the methods of the presently disclosed subject matter.

In some embodiments of the presently disclosed subject matter, a kit is provided for determining the probability of the presence of lung cancer in a subject based on measuring an amount of each member of a panel of biomarkers in a sample of the subject, the kit comprising: i) detection molecules specific for each of the biomarkers in the panel, wherein the biomarkers comprise at least two of the proteins selected from the group consisting of alpha-1-antitrypsin, carcinoembryonic antigen, squamous cell carcinoma antigen, retinol binding protein, transferrin, and haptoglobin, and ii) directions for measuring the amount of each member of the panel of biomarkers and determining the probability of lung cancer. The phrase "detection molecule" is used herein in its broadest sense to include any molecule that can bind with sufficient specificity to one of the members of the biomarker panel to allow for detection of the particular biomarker member in the presence or absence of the other members of the panel. To allow for detection can mean to determine the presence or absence of the particular biomarker member and, in some embodiments, can mean to determine the amount of the particular biomarker. Detection molecules can include antibodies and antibody fragments. In some embodiments, the detection molecules comprise a conjugated detectable group. In some embodiments, the detection molecules comprise antibodies specific for each of the protein biomarkers in the panel.

Approaches for producing a detectable signal include the use of radioactive labels (e.g., ³⁵S ¹²⁵I, ¹³¹I), fluorescent labels, enzyme labels (e.g., horseradish peroxidase, alkaline phosphatase), fluorescent labels (e.g., fluorescein) and so forth, in accordance with known techniques, as will be apparent to one skilled in the art upon review of the present disclosure. Many methods are known in the art for detecting binding in an immunoassay and are within the scope of the presently disclosed subject matter.

In some embodiments, direct detection methods are provided, such as, for example, wherein the detection molecule is a primary antibody specific for a member of the biomarker panel and detection is by using a label on the primary antibody. In some embodiments, the detection molecule can be detected using an indirect method such as by detecting binding of a specific binding partner to the detection molecule. The specific binding partner can be any molecule that binds with sufficient specificity to the detection molecule to allow for detection of the particular detection molecule in the presence or absence of the detection molecules for the other members of the biomarker panel. In some embodiments, the detection molecule is a primary antibody and the primary antibody can be detected by detecting binding of a secondary antibody or a reagent or other specific binding partner to the primary antibody. For example, in some embodiments the specific binding partner can be a secondary antibody that recognizes the detection molecule that is a primary antibody. In some embodiments the specific binding partner can be a molecule that specifically binds to a group on the detection molecule such as, for example, a biotin group on the detection molecule. In some embodiments, the binding partner can be labeled. In some embodiments, the binding partner is a secondary antibody that can be labeled.

For example, in some embodiments, indirect detection methods can involve a detection molecule that is an unlabeled primary antibody and a binding partner that is a labeled secondary antibody. This method can be more sensitive than direct detection methods due to signal amplification through more than one secondary antibody reaction with different antigenic sites on the primary antibody. In some embodiments, the indirect detection method is an immunofluorescence method, wherein the secondary antibody can be labeled with a fluorescent dye such as FITC, rhodamine or Texas red. In some embodiments, the indirect detection method is an immunoenzyme method, wherein the secondary antibody can be labeled with an enzyme such as peroxidase, alkaline phosphatase or glucose oxidase.

In some embodiments, an immunoassay can comprise antibodies specific for each of the members of the panel of protein biomarkers and an approach for producing a detectable signal. In some embodiments, the antibodies can be immobilized on a support (such as a bead, plate or slide) in accordance with known techniques and contacted with a test sample in liquid phase. The support can then be separated from the liquid phase and either the support phase or the liquid phase can be examined for the detectable signal that is related to the presence of the protein biomarker.

The presently disclosed subject matter includes kits for detecting each of the members of the panel of biomarkers. In some embodiments, the kit can comprise detection molecules, such as antibodies, specific for the protein biomarkers in the panel, the reagents necessary for producing a detectable signal as described above and buffers. In some embodiments, the kit can contain all of the components necessary to perform a detection assay, including all controls, directions for performing assays, and any necessary software for analysis and presentation of results.

Detection kits for carrying out the methods of the presently disclosed subject matter can be produced in a number of ways. In some embodiments, the detection kit can comprise a detection molecule that is an antibody or antibody fragment that specifically binds to a protein biomarker in the panel immobilized on a solid support, and a second antibody or antibody fragment specific for the first antibody or antibody fragment conjugated to a detectable group. In some embodiments, the kit can also include ancillary reagents such as buffering agents and protein stabilizing agents, and can include (where necessary) other members of the detectable signal-producing system of which the detectable group is a part (e.g., enzyme substrates); agents for reducing background interference in a test; control reagents; apparatus for conducting a test, and the like, as will be apparent to those skilled in the art upon a review of the instant disclosure.

In some embodiments, the detection kit can comprise antibodies or antibody fragments specific for each of the protein biomarkers in the panel, and a specific binding partner for each of the antibodies that is conjugated to a detectable group. Ancillary agents as described above can likewise be included. The test kit can be packaged in any suitable manner, typically with all groups in a single container along with a sheet or printed instructions for carrying out the test.

In some embodiments, the detection assay for the biomarker panels can be automated. Methods for the automation of immunoassays include those described in U.S. Pat. Nos. 5,885,530, 4,981,785, 6,159,750, and 5,358,691, each of which is herein incorporated by reference. In some embodiments, the analysis and presentation of results can also be automated. In this manner, a clinician can access the test results using any suitable approach or device. Thus, in some embodiments, a clinician need not understand the raw data, as the data can be presented directly to the clinician in its most useful form. The clinician is then able to immediately utilize the information to optimize care of the subject. The presently disclosed subject matter provides any method capable of receiving, processing, and transmitting the information to and from laboratories conducting the assays, information providers, medical personnel, and subjects.

### III. EXAMPLES

The presently disclosed subject matter will now be described more fully hereinafter with reference to the accompanying Examples, in which representative embodiments are shown. The presently disclosed subject matter can, however, be embodied in different forms and should not be construed as limited to the embodiments set forth herein. Rather, these embodiments are provided so that this disclosure will be thorough and complete, and will fully convey the scope of the embodiments to those skilled in the art.

### Example 1

### Selection of Candidates for the Biomarker Panel

Two different platforms were used to detect differentially expressed serum proteins from patients with or without lung cancer, with the aim of capitalizing on the tendency of each platform to identify groups of proteins possessing different physicochemical properties. The two different methodologies that were used to highlight serum proteins that are expressed differentially between patients with lung cancer versus age- and gender-matched individuals without lung cancer were 2-dimensional difference gel electrophoresis (2D-DIGE) and Matrix-assisted laser desorption/ionization-time of flight mass spectrometry (MALDI-TOF MS). All sera were collected under a protocol approved by the Duke University Institutional Review Board (IRB) and all patients provided written informed consent.

2D-DIGE: For the 2D-DIGE study, serum proteins from 10 patients with newly diagnosed non-small cell lung cancer (NSCLC) were compared to those from 10 individuals without cancer. Prior to 2D-DIGE analysis, serum samples (10 µl each) from 10 patients with newly diagnosed NSCLC and from 10 individuals without cancer were depleted of six abundant proteins using a VivaPure SEPPRO Mixed 6 Kit (VIVASCIENCE, Edgewood, New York, United States of America) according to the manufacturer's instructions. Depleted serum samples were diluted into buffer consisting of 8 M urea, 2 M thiourea, 20 mM Tris-HCl, pH 8.5, and 4% (w/v) CHAPS and the proteins labeled with Cy3 or Cy5. Dye labeling efficiency was normalized by randomly assigning 5 cancer and 5 control sera to Cy3 and the remaining 5 cancer and 5 non-cancer sera to Cy5. A pooled internal standard, consisting of equal volumes of each of the 20 depleted serum samples, was labeled with Cy2. Equal amounts of protein from all 3 samples (150 µg each cancer, control, and pool) were then mixed and subjected to 2D-gel electrophoresis using 13 cm IPG strips (AMERSHAM BIOSCIENCES, Piscataway, New Jersey, United States of America), pH 3-10, for the first dimension and 12% (w/v) polyacrylamide gels for the second dimension.

The gels were imaged on a TYPHOON 9400 Variable Mode Imager (Amersham) at the 3 excitation and emission wavelengths specific for each of the CyDyes. All gels were analyzed using DECYDER 5.0 (Amersham) software. After the gel images were cropped to exclude peripheral gel artifacts arising from the IPG strip and the dye front, intra-gel analysis was performed using DECYDER Difference In-gel Analysis software. Approximately 2000 spots were auto-detected in each gel and these were subsequently filtered with manually selected filter exclusion parameters. Spot maps of each filtered gel were saved and imported into BIOLOGICAL VARIATION ANALYSIS software for inter-gel matching and statistical analyses. Using an independent student's T-Test to score differences in spot intensity between the cancer and control sera, protein spots that were found to have a T-score of 0.1 or less were excised and submitted to the UNC-Duke Proteomics Center (Chapel Hill, North Carolina) for protein identification.

Analysis of the DIGE gels revealed four (4) proteins (transferrin, fibrinogen beta chain, retinol binding protein (RBP), and haptoglobin) whose levels differed by at least 1.5-fold between the two groups (p ≤ 0.05) (See Table 1). Fibrinogen beta chain was excluded from use in the biomarker panel since its involvement in coagulation and status as an acute phase protein would likely influence its concentration in serum irrespective of the presence of lung cancer (6).

**Table 1**

| Proteins Identified by 2D-DIGE | | | | | | | | |
|---|---|---|---|---|---|---|---|---|
| Protein Name | Gene Name | Accession No.* | MW (Da) | Peptide Count | MS and MS/S Score | Peptide Ion Score | Average Ratio | P Value |
| Transferrin | TF | P02787 | 77050 | 13 | 154 | 84 | -1.5 | 0.043 |
| Fibrinogen beta chain | FGB | P02675 | 55928 | 14 | 220 | 125 | -2.55 | 0.017 |
| RBP | RBP4 | P02753 | 23010 | 5 | 117 | 83 | -1.85 | 0.01 |
| Haptoglobin | HP | P00738 | 45205 | 5-8 | 176-224 | 103-122 | 1.65-1.83 | 0.009-0.038 |

MALDI-TOF: For the MALDI-TOF MS experiment, serum samples from 18 NSCLC patients and 18 control individuals were first subjected to solution-phase isoelectric focusing (IEF) using a ROTOFOR Cell (Bio-Rad, Hercules, California, United States of America), which fractionates each sample into 20 tubes. Each fraction was then analyzed by MALDI-TOF MS. Prior to IEF, 2 ml of each serum sample was depleted of salt by overnight dialysis (7000 MWCO) against distilled, deionized water (DD water) and then centrifuged to remove insoluble material. The dialyzed serum was diluted to 18 ml with DD water and carrier ampholytes (pH 3 - 10 range) were added to a final concentration of 2% (w/v). Isoelectric focusing was carried out at 12 W constant power using a ROTOFOR PREP IEF Cell (Bio-Rad) according to the manufacturer's instructions. At the completion of the ROTOFOR run, 20 fractions (0.5-1.5 ml each; average protein concentration ∼1.5 mg/ml) were harvested into microcentrifuge tubes and stored at -80° C until use.

MALDI-TOF MS analysis of IEF-fractionated serum was carried out using a conventional dried droplet protocol using a saturated solution of sinapinic acid (SA; Sigma Chemical Company, St. Louis, Missouri, United States of America) in 45% (v/v) acetonitrile and 0.1% (v/v) TFA as the matrix. SA was obtained in crystalline form and was used without further purification. One microliter of each ROTOFOR fraction was diluted with 5 µl SA matrix solution and 1.2 µl of the resulting mixture deposited on the MALDI sample stage. External calibration of MALDI-TOF mass spectra was achieved by using a preset calibration equation that was defined in separate, but similar, MALDI-TOF MS experiments on proteins of known molecular weight.

All MALDI-TOF-MS mass spectra were acquired on a Voyager DE-PRO Biospectrometry Workstation (PerSeptive Biosystems, Framingham, Massachusetts, United States of America) in the linear mode using a nitrogen laser (337 nm). Mass spectra were collected in the positive-ion mode using an acceleration voltage of 25 kV and a delay of 475 ns. The grid voltage, guide wire voltage, and low mass gate were set to 94.0%, 0.15% and 1000.0 m/z, respectively.

Spectra generated from each of the 20 fractions for each sample were then combined to form 36 composite spectra (7). Comparison of NSCLC spectra with control spectra found a statistically significant peak at m/z 50430 that was differentially represented in NSCLC serum compared to control serum. The protein was identified as alpha-1-antitrypsin (AAT) by partial purification, 2D-gel electrophoresis and MALDI-TOF peptide mass fingerprinting and MS/MS sequencing. AAT has been associated with both the detection and etiology of lung cancer (8, 9).

Two well-studied cancer biomarkers, carcinoembryonic antigen (CEA) and squamous cell carcinoma antigen (SCC) were also included in the biomarker panel. Neither of these biomarkers has been demonstrated to be effective by itself in lung cancer diagnosis or treatment follow-up (10, 11).

### Example 2

### Patient Samples

All serum samples were selected from the IRB-approved repository. The samples were all collected, processed, and stored in a similar fashion. Serum samples were selected from 99 sequential patients with a new diagnosis of lung cancer and who had not had previous treatment. Serum had been drawn from the patients at the time of initial diagnosis. In addition, 98 serum samples were selected from age and gender matched control patients without cancer who had been seen in the same general university practice in the same time period. Patient demographics and clinical profiles are shown in Table 2. Fifty serum samples each from the cancer and control groups were used to develop a model that was validated using 49 serum samples from the cancer group and 48 from the control group. Clinical data including past medical history, medications, stage at diagnosis, histology and outcome were available on each patient whose serum was used for the study.

**Table 2**

| Patient Demographics and Clinical Profiles | | | | |
|---|---|---|---|---|
| **Training Set** | **Test Set** | | | |
| Cancer (n = 50) | Control (n = 50) | Cancer (n = 49) | Control (n = 49) | |
| Age (y) | 68.5 ± 9.7 | 63.33 ± 16.76 | 67.16 ± 11.26 | 56.04 ± 15.11 |
| Range (y) | 47 - 91 | 23 - 93 | 34 - 91 | 24 - 87 |
| Gender | | | | |
| Male | 29 | 28 | 27 | 23 |
| Female | 21 | 22 | 22 | 26 |
| Stage | | | | |
| I | 20 | 16 | | |
| II | 2 | 3 | | |
| III | 15 | 19 | | |
| IV | 13 | 11 | | |
| Histology | | | | |
| Adenocarcinoma | 16 | 22 | | |
| Adenosquamous cell carcinoma | 1 | 0 | | |
| Bronchioloalveolar carcinoma | 0 | 4 | | |
| Squamous cell carcinoma | 13 | 9 | | |
| Large cell carcinoma | 1 | 0 | | |
| Small cell carcinoma | 0 | 4 | | |
| Non-small cell carcinoma, unspecified | 19 | 10 | | |

### Example 3

### Biomarker Assays

The serum levels of all proteins constituting the biomarker panel (i.e. transferrin, RBP, haptoglobin, AAT, CEA, and SCC) were determined in all specimens in the training and validation sets using commercially available enzyme-linked immunosorbent assays (ELISAs) according to the manufacturers' instructions (See Table 3). Assays for each biomarker were conducted simultaneously for both the training and validation sets to eliminate fluctuations in the data due to different freeze-thaw cycles or other sources of day-to-day assay variability.

**Table 3**

| Biomarker Panel ELISAs | | | |
|---|---|---|---|
| **Biomarker** | **Vendor** | **Location** | **Catalog Number** |
| CEA | United Biotech | Mountain View, CA, USA | CM-201 |
| SCC | Can-Ag | Gothenburg, Sweden | 800-10 |
| Transferrin | ADI | San Antonio, TX, USA | 1210 |
| RBP | AssayPro | St. Charles, MO, USA | ER1005-1 |
| AAT | ALPCO | Salem, NH, USA | 30-6750 |
| Haptoglobin | Invitrogen | Carlsbad, California, USA | KHA0022 |

### Example 4

### Data Analysis

A tree-structured data analytic technique referred to as Classification and Regression Tree (CART) Analysis was used to classify individuals as either having lung cancer or not having lung cancer, based on the serum levels of the proteins in the biomarker panel (12, 13). The CART software (Salford Systems, San Diego, California, United States) used a Gini splitting algorithm with 1 0-fold cross-validation that favored even splits and would not allow splits of nodes with 5 or fewer observations. The CART model was developed using the ELISA serum data obtained from the 50 sequential patients with lung cancer who had serum drawn at the time of initial diagnosis (and who had no previous treatment), and from the 50 patients without cancer matched for age and gender (See Example 2). These 100 samples constituted the training dataset. The model binned each sample into a terminal node with an associated probability of lung cancer or of not having lung cancer.

### Example 5

### Training Set Selection of a Panel of 4 Serum Markers

Since the goal of the current study was to identify a panel of proteins whose serum levels could be used for early detection of lung cancer, a CART model was trained to classify 100 serum specimens as being from individuals with or without lung cancer. In the training set, CART analysis identified CEA, RBP, SCC, and AAT as a panel with 7 terminal nodes (See Figure 1). Overall, the tree correctly classified 44/50 (88%) of lung cancer sera and 41/50 (82%) of non-cancer sera (sensitivity 89.3%, specificity 84.7%). Sixty-eight percent (34/50) of all lung cancer cases were binned in terminal nodes 4, 5 and 7, while only 6% (3/50) of control cases were in these nodes. Therefore, if a patient was assigned to one of these terminal nodes, there was a 92% (34/37) probability that they had lung cancer. The probability of lung cancer for each of the 7 terminal nodes is shown in Table 4.

**Table 4**

| Classification of Patients by CART Analysis | | | | | | | | |
|---|---|---|---|---|---|---|---|---|
| **Training Set** | **Test Set** | | | | | | | |
| Terminal Node | *Class | Determinants | n | Cancer (%) | No Cancer (%) | n | Cancer (%) | No Cancer (%) |
| 1 | 2 | ^{†} CEA ≤ 1.01 | 22 | 3 (13.6) | 19 (86.4) | 24 | 4 (16.7) | 20 (83.3) |
| 2 | 1 | 1.01 < CEA ≤ 2.14 SCC ≤ 0.77 | 16 | 10 (62.5) | 6 (37.5) | 20 | 7 (35) | 13 (65) |
| 3 | 2 | 1.01 < CEA ≤ 2.14 SCC > 0.77 AAT ≤ 2.04 | 19 | 2 (10.5) | 17 (89.5) | 15 | 7 (46.7) | 8 (53.3) |
| 4 | 1 | 1.01 < CEA ≤ 2.14 SCC > 0.77 AAT > 2.04 | 5 | 4 (80) | 1 (20) | 10 | 7 (70) | 3 (30) |
| 5 | 1 | CEA > 2.14 RBP ≤ 102.9 | 15 | 15 (100) | 0 (0) | 8 | 8 (100) | 0 (0) |
| 6 | 2 | 2.14 < CEA ≤ 3.47 RBP > 102.9 | 6 | 1 (16.7) | 5 (83.3) | 7 | 3 (42.9) | 4 (57.1) |
| 7 | 1 | CEA > 3.47 RBP > 102.9 | 17 | 15 (88.2) | 2 (11.8) | 13 | 13 (100) | 0 (0) |

### Example 6

### Test Set Validation of the Panel of 4 Serum Markers

The same classification tree as in Figure 1 was tested, without knowledge of true diagnosis, on an independent set of biomarker data from 97 patients, comprised of 49 serum samples from the patients with a new diagnosis of lung cancer and 48 age and gender matched controls (See Example 2). Each sample was assigned to a terminal node, each of which is associated with a probability of malignancy derived from the analysis of the training set. The classification tree correctly classified 35/49 (71.4%) lung cancer sera and 32/48 (66.7%) non-cancer control sera (sensitivity 77.8%, specificity 75.4%). Fifty-seven percent (28/49) of all lung cancer cases were binned in terminal nodes 4, 5 and 7 (Figure 1), while only 6% (3/48) of control cases were in these nodes. If a patient was assigned to one of these terminal nodes, they had a 90% (27/30) probability of having lung cancer. The probability of lung cancer for each of the 7 terminal nodes is shown in Table 4 above. Sixty-two percent (10/16) of stage I patients, 66% (2/3) of stage II patients, 63% (12/19) of stage III patients, and 100% of stage IV patients (11/11) were accurately assigned to a lung cancer node, as shown in Figure 2A. The distribution of histology according to the terminal nodes is shown in Figure 2B.

Although correct classification rates with the test set were lower overall than with the training set, individual nodes varied in their ability to classify sera in the test set. As shown in Table 4, Nodes 1, 5 and 7 performed equally well or better with the test set as compared to the training set. Together, these three nodes correctly classified 20/24 (83%) non-cancer sera and 21/25 (84%) cancer sera in the test set. Nodes 2, 3, 4, and 6 performed better with the training set, with correct classification rates for the test set ranging from 35 to 70%. As the most accurate nodes observed in both the training and test sets are determined by CEA levels alone or in combination with RBP. These two biomarkers can serve as the foundation of some embodiments of the presently disclosed lung cancer serum diagnostic tests.

### REFERENCES

1. Mountain, C. F. Revisions in the International System for Staging Lung Cancer. Chest, 111: 1710-1717., 1997.
2. Swensen, S. J., Jett, J. R., Hartman, T. E., Midthun, D. E., Mandrekar, S. J., Hillman, S. L., Sykes, A. M., Aughenbaugh, G. L., Bungum, A. O., and Allen, K. L. CT screening for lung cancer: five-year prospective experience. Radiology, 235: 259-265, 2005.
3. Sone, S., Li, F., Yang, Z. G., Honda, T., Maruyama, Y., Takashima, S., Hasegawa, M., Kawakami, S., Kubo, K., Haniuda, M., and Yamanda, T. Results of three-year mass screening programme for lung cancer using mobile low-dose spiral computed tomography scanner. Br J Cancer, 84: 25-32, 2001.
4. Manser, R., Dalton, A., Carter, R., Byrnes, G., Elwood, M., and Campbell, D. A. Cost-effectiveness analysis of screening for lung cancer with low dose spiral CT (computed tomography) in the Australian setting. Lung Cancer, 48: 171-185, 2005.
5. Mahadevia, P. J., Fleisher, L. A., Frick, K. D., Eng, J., Goodman, S. N., and Powe, N. R. Lung cancer screening with helical computed tomography in older adult smokers: a decision and cost-effectiveness analysis. Jama, 289: 313-322, 2003.
6. Gabay, C. and Kushner, I. Acute-phase proteins and other systemic responses to inflammation. N Engl J Med, 340: 448-454, 1999.
7. Wang, M. Z., Howard, B., Campa, M. J., Patz, E. F., Jr., and Fitzgerald, M. C. Analysis of human serum proteins by liquid phase isoelectric focusing and matrix-assisted laser desorption/ionization-mass spectrometry. Proteomics, 3: 1661-1666, 2003.
8. Ljujic, M., Nikolic, A., Divac, A., Djordjevic, V., and Radojkovic, D. Screening of alpha-1-antitrypsin gene by denaturing gradient gel electrophoresis (DGGE). J Biochem Biophys Methods, 68: 167-173, 2006.
9. Zelvyte, I., Wall mark, A., Piitulainen, E., Westin, U., and Janciauskiene, S. Increased plasma levels of serine proteinase inhibitors in lung cancer patients. Anticancer Res, 24: 241-247, 2004.
10. Molina, R., Agusti, C., Mane, J. M., Filella, X., Jo, J., Joseph, J., Gimenez, N., Estape, J., and Ballesta, A. M. CYFRA 21-1 in lung cancer: comparison with CEA, CA 125, SCC and NSE serum levels. Int J Biol Markers, 9: 96-101, 1994.
11. Schneider, J. Tumor markers in detection of lung cancer. Adv Clin Chem, 42: 1-41, 2006.
12. Breiman, L., Friedman, J., Olshen, R., Stone, C. Classification and Regression Trees. Pacific Grove, CA: Wadsworth, 1984.
13. Steinberg, D., Golovnya, M., and Tolliver, D. In: CART for Windows User Guide. San Diego, CA: Salford Systems, 2002.
14. Tas, F., Aydiner, A., Topuz, E., Yasasever, V., Karadeniz, A., and Saip, P. Utility of the serum tumor markers: CYFRA 21.1, carcinoembryonic antigen (CEA), and squamous cell carcinoma antigen (SCC) in squamous cell lung cancer. J Exp Clin Cancer Res, 19: 477-481, 2000.
15. Kulpa, J., Wojcik, E., Reinfuss, M., and Kolodziejski, L. Carcinoembryonic antigen, squamous cell carcinoma antigen, CYFRA 21-1, and neuron-specific enolase in squamous cell lung cancer patients. Clin Chem, 48: 1931-1937, 2002.
16. Marcus, P. M., Bergstralh, E. J., Fagerstrom, R. M., Williams, D. E., Fontana, R., Taylor, W. F., and Prorok, P. C. Lung cancer mortality in the Mayo Lung Project: impact of extended follow-up. J Natl Cancer Inst, 92: 1308-1316, 2000.
17. Bach, P. B., Jett, J. R., Pastorino, U., Tockman, M. S., Swensen, S. J., and Begg, C. B. Computed tomography screening and lung cancer outcomes. Jama, 297: 953-961, 2007.
18. Henschke, C. I., Yankelevitz, D. F., Libby, D. M., Pasmantier, M. W., Smith, J. P., and Miettinen, O. S. Survival of patients with stage I lung cancer detected on CT screening. N Engl J Med, 355: 1763-1771, 2006.
19. Patz, E. F., Jr., Goodman, P. C., and Bepler, G. Screening for lung cancer. N Engl J Med, 343: 1627-1633, 2000.
20. Patz, E. F., Jr. Lung cancer screening, overdiagnosis bias, and reevaluation of the Mayo Lung Project. J Natl Cancer Inst, 98: 724-725, 2006.
21. Petersen, R. P., Campa, M. J., Sperlazza, J., Conlon, D., Joshi, M. B., Harpole, D. H., Jr., and Patz, E. F., Jr. Tumor infiltrating Foxp3+ regulatory T-cells are associated with recurrence in pathologic stage I NSCLC patients. Cancer, 107: 2866-2872, 2006.
22. Zhong, L., Coe, S. P., Stromberg, A. J., Khattar, N. H., Jett, J. R., and Hirschowitz, E. A. Profiling tumor-associated antibodies for early detection of non-small cell lung cancer. J Thorac Oncol, 1: 513-519, 2006.
23. Welsh, T. J., Green, R. H., Richardson, D., Waller, D. A., O'Byrne, K. J., and Bradding, P. Macrophage and mast-cell invasion of tumor cell islets confers a marked survival advantage in non-small-cell lung cancer. J Clin Oncol, 23: 8959-8967, 2005.
24. Condeelis, J. and Pollard, J. W. Macrophages: obligate partners for tumor cell migration, invasion, and metastasis. Cell, 124: 263-266, 2006.

It will be understood that various details of the presently disclosed subject matter may be changed without departing from the scope of the presently disclosed subject matter. Furthermore, the foregoing description is for the purpose of illustration only, and not for the purpose of limitation.

### Preferred Embodiments of the invention

1. A method for assigning a subject to a group having a higher or lower probability of lung cancer, the method comprising:
   a) determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; and
   b) assigning the subject to a group having a higher or lower probability of lung cancer based on the determined amount of each biomarker member of the panel.
2. The method of Embodiment 1, wherein the panel of biomarkers comprises at least three of the proteins alpha-1-antitrypsin, carcinoembryonic antigen, squamous cell carcinoma antigen, retinol binding protein, transferrin and haptoglobin.
3. The method of Embodiment 1, wherein the panel of biomarkers comprises at least four of the proteins alpha-1-antitrypsin, carcinoembryonic antigen, squamous cell carcinoma antigen, retinol binding protein, transferrin and haptoglobin.
4. The method of Embodiment 1, wherein the panel of biomarkers comprises at least the proteins alpha-1-antitrypsin, carcinoembryonic antigen, squamous cell carcinoma antigen and retinol binding protein.
5. The method of Embodiment 1, wherein the panel of biomarkers comprises at least the proteins alpha-1-antitrypsin, carcinoembryonic antigen, squamous cell carcinoma antigen, retinol binding protein, transferrin, and haptoglobin.
6. The method of Embodiment 1, comprising applying a predetermined algorithm to the determined amount of each biomarker in the panel; and using the results of the algorithm to assign the subject to the group having a higher or lower probability of lung cancer.
7. The method of Embodiment 6, wherein the algorithm is a decision tree analysis.
8. The method of Embodiment 6, wherein the algorithm is a Classification and Regression Tree analysis.
9. The method of Embodiment 1, wherein the sample is a serum sample.
10. The method of Embodiment 1, wherein the subject is a human subject.
11. A method for managing treatment of a subject with potential lung cancer, the method comprising:
   a) determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin;
   b) assigning the subject to a group having a higher or lower probability of lung cancer based on the determined amount of each biomarker in the panel; and
   c) managing the treatment of the subject with potential lung cancer based on the group to which the subject is assigned.
12. A method for molecular staging of a lung tumor or suspected lung tumor, the method comprising:
   a) determining an amount of each member of a panel of biomarkers in a sample from a subject having a tumor or suspected tumor, wherein the panel of biomarkers comprises at least two of the proteins alpha-1-antitrypsin, carcinoembryonic antigen, squamous cell carcinoma antigen, retinol binding protein, transferrin, or haptoglobin;
   b) assigning the subject to a group having a higher or lower probability of a stage of lung tumor or suspected lung tumor based on the determined amount of each biomarker in the panel; and
   c) determining the molecular stage of the tumor or suspected tumor based on the group to which the subject is assigned.
13. A method for assigning a subject to a high-risk group for lung cancer, the method comprising:
   a) determining an amount of each member of a panel of biomarkers in a sample from the subject, wherein the panel of biomarkers comprises at least two of the proteins alpha-1-antitrypsin, carcinoembryonic antigen, squamous cell carcinoma antigen, retinol binding protein, transferrin, and haptoglobin; and
   b) assigning the subject to a group having a high-risk of lung cancer based on the determined amount of each biomarker in the panel.
14. A kit for determining the probability of the presence of lung cancer in a subject based on measuring an amount of each member of a panel of biomarkers in a sample of the subject, the kit comprising: i) detection molecules specific for each of the biomarkers in the panel, wherein the biomarkers comprise at least two of the proteins selected from the group consisting of alpha-1-antitrypsin, carcinoembryonic antigen, squamous cell carcinoma antigen, retinol binding protein, transferrin, and haptoglobin, and ii) directions for measuring the amount of each member of the panel of biomarkers and determining the probability of lung cancer.
15. The kit of Embodiment 14, wherein the detection molecules comprise a conjugated detectable group.
16. The kit of Embodiment 14, wherein the detection molecules specific for the protein biomarkers in the panel are immobilized on a solid support.
17. The kit of Embodiment 14, wherein the detection molecules comprise antibodies specific for each of the protein biomarkers in the panel.
18. The kit of Embodiment 17, comprising a second specific antibody for each of the antibodies specific for the protein biomarkers, wherein the second specific antibody is conjugated to a detectable group.
19. The kit of Embodiment 14, comprising a specific binding partner for each of the detection molecules specific for the biomarkers in the panel, wherein each specific binding partner is conjugated to a detectable group.
20. The kit of Embodiment 15, 18 or 19, wherein the detectable group is selected from the group consisting of radioactive labels, fluorescent labels and enzyme labels.
21. The kit of Embodiment 14, comprising one or more of buffering agents, protein stabilizing agents, enzyme substrates, background reducing agents, control reagents, an apparatus for conducting the detection, and any necessary software for analysis and presentation of results.
22. The kit of Embodiment 14, wherein the sample is a serum sample from a human subject.
23. The kit of Embodiment 14, wherein the biomarkers comprise at least three of the proteins selected from the group consisting of alpha-1-antitrypsin, carcinoembryonic antigen, squamous cell carcinoma antigen, retinol binding protein, transferrin, and haptoglobin.
24. The kit of Embodiment 14, wherein the biomarkers comprise at least four of the proteins selected from the group consisting of alpha-1-antitrypsin, carcinoembryonic antigen, squamous cell carcinoma antigen, retinol binding protein, transferrin, and haptoglobin.
25. The kit of Embodiment 14, wherein the biomarkers comprise at least the proteins alpha-1-antitrypsin, carcinoembryonic antigen, squamous cell carcinoma antigen and retinol binding protein.
26. The kit of Embodiment 14, wherein the biomarkers comprise at least the proteins alpha-1-antitrypsin, carcinoembryonic antigen, squamous cell carcinoma antigen, retinol binding protein, transferrin, and haptoglobin.

## Claims

1. A method for assigning a subject, preferably a human, to a group having a higher or lower probability of having lung cancer, the method comprising:
a) determining an amount of each member of a panel of biomarkers in a sample, preferably a serum sample, from the subject, wherein the panel of biomarkers comprises at least carcinoembryonic antigen, squamous cell carcinoma antigen, and alpha-1-antitrypsin; and
b) assigning the subject to a group having a higher or lower probability of having lung cancer based on the determined amount of each biomarker member of the panel.

2. The method of Claim 1, wherein the panel of biomarkers comprises additionally at least one of the proteins retinol binding protein, transferrin, and haptoglobin.

3. The method of any one of Claims 1-2, comprising applying a predetermined algorithm to the determined amount of each biomarker in the panel; and using the results of the algorithm to assign the subject to the group having a higher or lower probability of having lung cancer.

4. The method of Claim 3, wherein the algorithm is a decision tree analysis or a Classification and Regression Tree analysis.

5. A method for molecular staging of a lung tumor or suspected lung tumor, the method comprising:
a) determining an amount of each member of a panel of biomarkers in a sample from a subject having a tumor or suspected tumor, wherein the panel of biomarkers comprises at least carcinoembryonic antigen, squamous cell carcinoma antigen, and alpha-1-antitrypsin;
b) assigning the subject to a group having a higher or lower probability of a stage of lung tumor or suspected lung tumor based on the determined amount of each biomarker in the panel; and
c) determining the molecular stage of the tumor or suspected tumor based on the group to which the subject is assigned.

6. The method of Claim 5, wherein the panel of biomarkers additionally comprises retinol binding protein, transferrin, or haptoglobin.

7. A method for assigning a subject to a high-risk group for having lung cancer, the method comprising:
a) determining an amount of each member of a panel of biomarkers in a sample from the subject, wherein the panel of biomarkers comprises at least carcinoembryonic antigen, squamous cell carcinoma and alpha-1-antitrypsin; and
b) assigning the subject to a group having a high-risk of having lung cancer based on the determined amount of each biomarker in the panel.

8. The method of Claim 7, wherein the panel of biomarkers additionally comprises retinol binding protein, transferrin, or haptoglobin.

9. A kit for determining the probability of the presence of lung cancer in a subject based on measuring an amount of each member of a panel of biomarkers in a sample of the subject, which is preferably a serum sample of a human subject, the kit comprising: i) detection molecules specific for each of the biomarkers in the panel, wherein the biomarkers comprise at least carcinoembryonic antigen, squamous cell carcinoma antigen, and alpha-1-antitrypsin; and ii) directions for measuring the amount of each member of the panel of biomarkers and determining the probability of lung cancer.

10. The kit of Claim 9, wherein the detection molecules comprise a conjugated detectable group, preferably wherein the detactable group is selected from the group consisting of radioactive labels, fluorescent labels, and enzyme labels; or
wherein the detection molecules are immobilized on a solid support.

11. The kit of Claim 9, wherein the detection molecules comprise antibodies specific for each of the protein biomarkers in the panel, optionally wherein the kit further comprises a second specific antibody for each of the antibodies specific for the protein biomarkers, wherein the second specific antibody is conjugated to a detectable group, preferably wherein the detectable group is selected from the group consisting of radioactive labels, fluorescent labels, and enzyme labels.

12. The kit of Claim 9, comprising a specific binding partner for each of the detection molecules specific for the biomarkers in the panel, wherein each specific binding partner is conjugated to a detectable group, preferably wherein the detectable group is selected from the group consisting of radioactive labels, fluorescent labels, and enzyme labels.

13. The kit of Claim 9, comprising one or more of buffering agents, protein stabilizing agents, enzyme substrates, background reducing agents, control reagents, an apparatus for conducting the detection, and any necessary software for analysis and presentation of results.

14. The kit of Claim 9, wherein the biomarkers further comprise at least one of the proteins selected from the group consisting of retinol binding protein, transferrin, and haptoglobin.
